# FASCICULE DE BREVET EUROPEEN

(11) **EP 3 155 095 B1**
(45) Date de publication et mention de la délivrance du brevet: **19.12.2018**
(21) Numéro de dépôt: 15733838.5
(22) Date de dépôt: 15.06.2015
(51) Int. Cl.: C12N 9/26

(54) **PROCEDE DE FABRICATION D'UNE SOLUTION AQUEUSE STABLE DE BÊTA-AMYLASE, SOLUTION AQUEUSE OBTENUE ET SES UTILISATIONS**
VERFAHREN ZUR HERSTELLUNG EINER STABILEN WÄSSRIGEN LÖSUNG VON BETA-AMYLASE, ERHALTENE WÄSSRIGE LÖSUNG UND VERWENDUNGEN DAVON
METHOD FOR MANUFACTURING A STABLE AQUEOUS SOLUTION OF BETA-AMYLASE, AQUEOUS SOLUTION OBTAINED AND USES THEREOF

(30) Priorité: 16.06.2014 FR 1455466
(43) Date de publication de la demande: 19.04.2017
(73) Titulaire: Roquette Frères, 62136 Lestrem (FR)
(72) Inventeur: COURBOIS, Vincent, 62400 Bethune (FR); LECOCQ, Aline, 62400 Bethune (FR); DUFLOT, Pierrick, 62136 La Couture (FR)
(74) Mandataire: Cabinet Plasseraud
(86) Numéro de dépôt international: PCT/FR2015/051581
(87) Numéro de publication internationale: WO 2015/193601

(56) Documents cités:
- WO-A1-2014/023915
- CN-A- 102 399 763
- US-A1- 2011 159 002
- TOLAN J S ET AL: "Cellulase from Submerged Fermentation", ADVANCES IN BIOCHEMICAL ENGINEERING, BIOTECHNOLOGY, SPRINGER, BERLIN, DE, vol. 65, 4 mai 1999 (1999-05-04), pages 41-67, XP008122759, ISSN: 0724-6145, DOI: 10.1007/3-540-49194-5 [extrait le 2003-06-30]

## Description

La présente invention est relative à un procédé de fabrication d'une solution aqueuse de β-amylase, notamment par mise en oeuvre de glycérol, de sorbate de potassium et de carbonate de sodium. Ce cocktail d'additifs s'avère particulièrement performant en vue de maintenir l'activité enzymatique de la β-amylase au cours du temps.

Un autre objet de la présente invention consiste en l'utilisation dudit cocktail avec la fonction particulière de maintien de l'activité enzymatique de la β-amylase. Un autre objet de la présente invention consiste en une solution aqueuse de β-amylase contenant le cocktail précité. Un dernier objet de la présente invention consiste en l'utilisation de ladite solution aqueuse de β-amylase en panification, en malterie, comme additif alimentaire, comme agent digestif, pour la production d'édulcorants, en pharmacie et enfin pour la production de maltose et de sirops enrichis en maltose.

Les β-amylases sont des exo-hydrolases qui libèrent des unités maltose à partir des β-extrémités non réductrices des polymères ou oligomères de glucose liés en α 1→4, la réaction s'arrêtant au premier point de branchement α 1→6 rencontré. Composants majoritaires du « pouvoir diastasique » (correspondant aux activités combinées des α-amylases, β-amylases, α-glucosidases et enzymes débranchantes) durant le maltage (germination artificielle des graines de céréales), les activités β-amylasiques isolées de ce cocktail enzymatique sont essentielles pour la production du maltose ou d'autres sucres fermentescibles générés à partir d'amidon.

L'activité saccharifiante des seules β-amylases est donc exploitée dans bon nombre d'applications : en panification, en malterie, comme additif alimentaire, voire même comme agent digestif, pour la production d'édulcorants, en pharmacie pour la production de vaccins, et enfin pour la production de maltose et de sirops enrichis en maltose (précurseur du maltitol et des sirops de maltitol).

Les procédés de fabrication de β-amylases sont nombreux. On sait ainsi que les graines d'orge, de seigle ou de blé non germées sont autant de matériels biologiques de choix pour la préparation commerciale, à grande échelle, de β-amylases. Il est par ailleurs connu de l'homme du métier que la moitié des β-amylases extractibles des graines non germées de l'orge, du blé ou du seigle peut être facilement obtenue sous la forme d'enzymes libres par extraction avec de l'eau et des solutions salines. L'autre moitié se présente en partie sous forme « liée » qui nécessite l'addition d'agents réducteurs ou des enzymes protéolytiques pour son extraction. Une autre fraction de β-amylases non directement extractible, dite « latente » a également été décrite : il faut des détergents pour l'extraire des graines de céréales. Par ailleurs, les procédés d'extraction de la β-amylase décrits dans l'état de la technique sont adaptés en fonction de l'application visée.

A cet égard, la Demanderesse a mis au point et protégé dans la Demande EP 2 414 379 un procédé original de production de β-amylases, en ce sens qu'il repose sur une matière première de départ jusqu'alors peu valorisée : les « fractions solubles ». Ces dernières étaient auparavant utilisées de manière exclusive comme source d'azote en fermentation et en tant qu'aliment nutritif pour le bétail une fois lesdites fractions enrichies en fibres.

De telles fractions solubles sont produites au cours de l'extraction par voie humide des composants des plantes amidonnières, comme le maïs, la pomme de terre, la patate douce, le blé, le riz, le pois, la fève, la fèverole, le manioc, le sorgho, le konjac, le seigle, le sarrasin et l'orge. Les composants dits « nobles », produits au cours de l'extraction, sont notamment les amidons, les protéines ou encore les fibres. Les « fractions solubles » désignent par opposition des constituants « non nobles » : il s'agit des résidus liquides issus de ladite extraction, quand bien même de tels résidus peuvent encore contenir sous forme de traces de rares substances insolubles ainsi que des particules et des colloïdes divers et variés.

Le procédé objet de la Demande EP 2 414 379 repose sur la sélection initiale de la fraction soluble à traiter, puis sur une étape de clarification réalisée par microfiltration, et enfin sur une étape de purification par ultrafiltration. A cette occasion, il a été démontré que la β-amylase obtenue était particulièrement bien adaptée à la préparation de sirops de maltose, au même titre qu'une β-amylase produite selon des techniques antérieures mais à partir de procédés plus complexes et plus onéreux.

Par la suite, la Demanderesse a également protégé des améliorations de ce procédé à travers la Demande de brevet FR 2 994 440 et la Demande de brevet français n° 13 56022 non encore publiée à la date de dépôt de la présente Demande. Ces améliorations reposent notamment sur l'utilisation de protéases au cours de l'étape de microfiltration, ce qui permet de réduire très fortement le colmatage des membranes de microfiltration et donc d'augmenter le temps de production avant lavage, ainsi que sur l'emploi de pectinases au cours de l'étape d'ultrafiltration, ce qui permet de limiter le colmatage des membranes d'ultrafiltration, de diminuer la viscosité du rétentat à l'issue de cette étape tout en augmentant sa richesse en β-amylase.

Or, le rétentat d'ultrafiltration qui contient la β-amylase sous forme concentrée est susceptible d'être conservé plusieurs semaines, voire plusieurs mois, avant d'être utilisé dans les applications qui ont déjà été citées. Il s'avère alors que son activité enzymatique va diminuer au fil du temps. On rappelle que l'activité d'une enzyme est par définition la quantité de substrat transformé (ou de produit formé) par unité de temps et dans les conditions de fonctionnement optimales de l'enzyme (température, de pH ...). Cette grandeur quantifie donc l'efficacité de l'enzyme.

Classiquement, la mesure de l'activité enzymatique est faite à travers la détermination d'un autre paramètre : l'activité diastasique. Cette dernière est exprimée en degré de pouvoir diastasique (°DP), défini comme la quantité d'enzyme contenue dans 0,1 ml d'une solution à 5 % en poids d'un échantillon de préparation d'enzymes suffisante pour réduire 5 ml de liqueur de Fehling, quand ledit échantillon est placé dans 100 ml du substrat pendant 1 h à 20°C.

On connaît aujourd'hui un certain nombre de documents décrivant des procédés d'obtention de β-amylase, en vue d'améliorer leur stabilité du point de vue de leur activité enzymatique.

Le document CN102965358 divulgue un procédé d'obtention à partir du soja d'une β-amylase par précipitation, puis drainage, clarification et ultrafiltration. Ledit procédé a recours à du chlorure de calcium, et optionnellement à des sels de l'acide sulfurique au niveau de l'étape de précipitation.

Le document CN102399763 décrit la production de β-amylase à partir de sons, avec ajout de chlorure de calcium et d'hydrogène phosphate de sodium, puis concentration et stabilisation en présence de sorbitol et de sorbate de potassium, avant stérilisation.

Le document CN101544967 divulgue un procédé de fabrication de β-amylases par précipitation, séparation et centrifugation, et préconise ensuite l'ajout de chlorure de calcium, d'acide orthophosphorique, de terres de diatomée et de glycérol.

Le document CN1225943 décrit un procédé de préparation de β amylase comprenant les étapes de d'ultrafiltration, concentration et précipitation d'extraits de poudre de soja, la précipitation étant précédée par l'ajout de sulfate de sodium et la régulation du pH entre 3,6 et 5.

Le document US 2,496,261 décrit un procédé d'obtention de β-amylase à partir de la patate douce, qui comprend une étape de précipitation en présence de sulfate d'ammonium puis acidification avec de l'acide chlorhydrique.

Le document US 4,024,000 décrit un procédé de préparation de β-amylase qui met en oeuvre des ions divalents ou trivalents choisis parmi les hydroxydes de calcium, magnésium, baryum, aluminium et leurs sels, et régulation du pH dans un intervalle compris entre 4,5 et 8.

Le document D1 Tolan et al « Cellulase from Submerged Fermentation », Advances in Biochemical Engineering, Vol.65, 4 mai 1999, Pages 41-67) mentionne que des compositions solides de cellulase peuvent être stabilisées pas l'ajout de carbonate de sodium. Il indique également que des compositions liquides peuvent être stabilisées par l'addition de glycérol et de sorbate de potassium.

Or, force est de constater qu'aucune de ces solutions ne permet d'obtenir une préparation de β-amylase sous forme d'une solution aqueuse qui soit suffisamment stable dans le temps, notamment sur des périodes de plusieurs semaines et plus particulièrement pendant au moins 70 jours, du point de vue de son activité enzymatique. Poursuivant ses travaux, la Demanderesse est parvenue à démontrer que seule une sélection bien particulière d'additifs permettait d'atteindre un tel objectif. Ce cocktail d'additifs est constitué de sorbate de potassium, de glycérol et de carbonate de sodium.

Aussi, un premier objet de l'invention consiste en un procédé de stabilisation d'une solution aqueuse de β-amylase obtenue à partir d'une fraction soluble de plante amidonnière, comprenant au moins une étape d'introduction dans ladite solution aqueuse de β-amylase de :
a) 0,05 à 0,5 % de sorbate de potassium ;
b) 30 à 50 % de glycérol ; et
c) 0,05 à 0,5 % de carbonate de sodium,
ces % étant exprimés en % en poids sec de chaque constituant par rapport au poids total de ladite solution aqueuse.

Avantageusement, le sorbate de potassium, le glycérol et le carbonate de sodium sont introduits dans ladite solution aqueuse de β-amylase dans les proportions suivantes :
a) de 0,1 à 0,3 %, et préférentiellement environ 0,2 % de sorbate de potassium ;
b) de 35 à 45 %, et préférentiellement environ 40 % de glycérol ;
c) de 0,1 à 0,3 %, et préférentiellement environ 0,2 % de carbonate de sodium ;
ces % étant exprimés en % en poids sec de chaque constituant par rapport au poids total de ladite solution aqueuse.

De manière avantageuse, la solution aqueuse de β-amylase présente une teneur en poids sec de β-amylase comprise entre 5 et 20 %, préférentiellement entre 10 % et 20 %, très préférentiellement d'environ 15 % par rapport au poids total de ladite solution aqueuse.

Le sorbate de potassium, le glycérol et le carbonate de sodium sont préférentiellement sous forme de solutions aqueuses. L'homme du métier saura adapter l'extrait sec de ces solutions, par rapport à la solubilité des produits mais aussi de manière à limiter la viscosité de ces solutions et de façon à le rendre aisément manipulable et notamment pompable.

Selon un mode de réalisation la solution aqueuse de β-amylase est obtenue par les étapes consistant à :
- fournir une fraction soluble de plantes amidonnières ;
- réaliser sur ladite fraction soluble une étape de microfiltration afin d'obtenir un perméat de microfiltration ;
- réaliser sur le perméat de microfiltration une étape d'ultrafiltration afin d'obtenir un rétentat d'ultrafiltration.
Dans ce mode de réalisation, c'est donc ledit rétentat d'ultrafiltration qui constitue ladite solution aqueuse de β-amylase, dans laquelle sont introduits le sorbate de potassium, le glycérol et le carbonate de sodium.

De manière plus détaillée et en amont du procédé conforme à l'invention, il convient de choisir la fraction soluble de plantes amidonnières à traiter. Cette sélection s'opère notamment dans le groupe constitué des fractions solubles de maïs, de pomme de terre, de patate douce, de blé, de riz, de pois, de fève, de fèverole, de manioc, de sorgho, de konjac, de seigle, de sarrasin et d'orge.

La microfiltration de la fraction soluble de plante amidonnière a notamment pour but d'éliminer les substances insolubles, les colloïdes, et le matériel microbiologique en vue d'obtenir une composition limpide contenant de la β-amylase. Cette dernière composition se trouve donc être le perméat de microfiltration. Selon une variante particulièrement avantageuse du présent mode de réalisation, ladite étape de microfiltration est effectuée en présence d'au moins une protéase. Au préalable de la microfiltration la protéase est mise en contact avec la fraction soluble de plante amidonnière à traiter : l'homme du métier saura adapter le temps de contact nécessaire à l'action de l'enzyme.

La protéase mise en oeuvre dans la présente invention est de préférence choisie parmi les sérine protéases, les protéases à thiol, les aspartyl protéases et les métallo protéases, et est plus particulièrement choisie parmi les métallo protéases. De manière nominative, les protéases préférées dans la présente invention sont les produits commercialisés sous les dénominations : Sumizyme™ APL, Lypaine™ 6500 L, Neutrase™ 0,8L, Brewlyve™ NP 900, Brewers Clarex™. On préfèrera utiliser une quantité de protéase comprise entre 0,01 % et 0,1 % en volume par rapport au volume de la fraction soluble de plante amidonnière à traiter.

L'étape de microfiltration du présent mode de réalisation est réalisée préférentiellement par microfiltration tangentielle membranaire. Plus particulièrement, la société Demanderesse recommande de réaliser la microfiltration tangentielle avec des membranes céramiques présentant une porosité de 0,1 µm à 1 µm.

De manière facultative, l'étape de microfiltration peut être précédée d'une étape de floculation des particules insolubles contenues dans la fraction soluble de plantes amidonnières, par toute technique connue par ailleurs de l'homme du métier.

Pour cette première étape de microfiltration, la Demanderesse recommande de travailler à un pH compris entre 4 et 5 et à une température comprise entre 40 °C et 50 °C.

L'étape de microfiltration est en particulier contrôlée par la montée de la pression transmembranaire (PTM) dans le temps, à débit de perméat fixé.

Dans le présent mode de réalisation, la microfiltration est suivie d'une étape d'ultrafiltration, visant tout d'abord à concentrer le perméat de microfiltration contenant la β-amylase, tout en le débarrassant d'éventuels sels résiduels contaminants, sucres et protéines. L'ultrafiltration est ainsi réalisée sur le perméat de la microfiltration de manière à obtenir un rétentat d'ultrafiltration contenant la β-amylase.

Plus particulièrement, la société Demanderesse recommande de réaliser l'ultrafiltration à l'aide de membranes présentant un seuil de coupure de 10 000 Da à 50 000 Da, de préférence un seuil de coupure de 30 000 Da. Les fractions solubles peuvent par exemple être ultrafiltrées sur un module équipé de membranes polysulfonées d'un seuil de coupure de 30 000 Da en cassettes à l'échelle laboratoire et membranes spiralées polysulfonées d'un seuil de coupure de 30 000 Da à l'échelle pilote. L'enzyme se concentre alors dans le rétentat au fil du temps.

Cette ultrafiltration peut être réalisée en présence de pectinase. Dans la présente Demande, le terme pectinase désigne des enzymes capables de décomposer les pectines qui sont des polymères de polysaccharide et qui sont l'un des constituants des parois cellulaires des plantes. Elles sont composées d'une chaîne principale d'acide uronique lié en 1-4. A ce titre, elles ne doivent pas être assimilées aux cellulases et aux hemicellulases auxquelles on les rattache par erreur : les cellulases sont des enzymes qui participent directement aux réactions de décomposition de la cellulose (chaînes linéaires de molécules de D-glucose) alors que les hemicellulases parviennent à hydrolyser l'hemicellulose (polymères branchés de sucres en général, tels que le glucose, xylose, etc...).

Typiquement, on introduit la pectinase dans le perméat de microfiltration avant de réaliser l'étape d'ultrafiltration et on la laisse agir.

L'homme du métier saura adapter le temps de contact nécessaire à l'action de l'enzyme. Typiquement, on laisse agir la pectinase de 30 minutes à 4 heures, préférentiellement de 30 minutes à 2 heures et ce, à une température comprise entre 25°C et 60°C, préférentiellement entre 25°C et 50°C.

Des pectinases particulièrement adaptées à la mise en oeuvre de la présente invention, sont les produits Rapidase™ ADEX D (pectinase ; DSM), Peclyve™ ESP (pectinase ; Lyven), ou Sumizyme™ ARS (pectinase et arabanase ; Takabio), sans pour autant que ces exemples ne soient limitatifs.

On préfèrera introduire une quantité de pectinase comprise entre 0,05 % et 1 % en volume par rapport au volume total du perméat de microfiltration.

L'étape d'ultrafiltration peut être suivie d'une étape de dialyse du rétentat d'ultrafiltration de façon à diminuer la concentration en impuretés dans ledit rétentat.

Par ailleurs, il est souhaitable de maintenir ladite solution de β-amylase telle qu'obtenue à une température inférieure à 15°C, préférentiellement inférieure à 10°C, idéalement aux environs de 5°C, de manière à améliorer encore le maintien de son activité enzymatique.

Un autre objet de la présente invention est l'utilisation, en vue de maintenir l'activité enzymatique de β-amylase en solution aqueuse, de :
a) sorbate de potassium ;
b) glycérol ; et
c) carbonate de sodium.

Par maintien de l'activité enzymatique, on entend la capacité à limiter la chute de degré DP° comme indiqué dans la partie expérimentale. Typiquement, on parlera de maintien si le degré DP° est toujours supérieur à au moins 70 % de sa valeur initiale, après 70 jours et à une température de 37°C.

Un autre objet de la présente invention consiste en une solution aqueuse de β-amylase stabilisée, contenant :
a) de 0,05 à 0,5 % de sorbate de potassium ;
b) de 30 à 50 % de glycérol ; et
c) de 0,05 à 0,5 % de du carbonate de sodium,
ces % étant exprimés en % en poids sec de chaque constituant par rapport au poids total de ladite solution aqueuse de β-amylase.

Plus particulièrement, cette solution aqueuse de β-amylase stabilisée contient :
a) de 0,1 à 0,3 %, et préférentiellement environ 0,2 % de sorbate de potassium ;
b) de 35 à 45 %, et préférentiellement environ 40 % de glycérol ;
c) de 0,1 à 0,3 %, et préférentiellement environ 0,2 % de carbonate de sodium ;
ces % étant exprimés en % en poids sec de chaque constituant par rapport au poids total de ladite solution aqueuse de β-amylase.

Elle présente également une teneur en poids sec de β-amylase comprise entre 5 et 20 %, préférentiellement entre 10 % et 20 %, très préférentiellement environ 15 % par rapport au poids total de ladite solution aqueuse.

Un dernier objet de la présente invention consiste en l'utilisation de la solution aqueuse de β-amylase stabilisée conforme à l'invention en panification, en malterie, comme additif alimentaire, comme agent digestif, pour la production d'édulcorants, en pharmacie pour la production de vaccins, et enfin pour la production de maltose et de sirops enrichis en maltose (précurseur du maltitol et des sirops de maltitol).

Les exemples qui suivent permettent de mieux appréhender l'invention, sans toutefois en limiter la portée.

### EXEMPLES

### Fabrication de solutions aqueuses de beta amylase

On commence par prélever en amidonnerie de blé une fraction soluble à l'entrée de l'évaporateur des solubles, étape classiquement mise en oeuvre pour fabriquer des produits destinés à l'alimentation du bétail, une fois concentrés. Ces produits sont commercialisés par la société Demanderesse sous le nom de Corami®. Ces fractions solubles présentent un pH compris entre 4 et 5 et une activité β-amylasique de l'ordre de 30 °DP / mL.

On réalise ici, sur un équipement à l'échelle pilote, la microfiltration de fractions solubles de blé. L'unité de microfiltration est équipée de membranes céramiques en oxyde de titane, dont le seuil de coupure est égal à 0,2 µm. Le débit de perméat est fixé à 12 L / (h m²). Le facteur de concentration volumique est égal à 1,5. La température et le pH du perméat sont respectivement égaux à 45°C et environ 4,5.

On ajoute dans la fraction soluble de la protéase Neutrase 0,8L (Novozyme), à une concentration fixée à 0,1 % en volume par rapport au volume total de ladite composition. On laisse au préalable agir cette protéase pendant 1 heure à température ambiante.

Puis on réalise une ultrafiltration telle que décrite ci-avant.

On obtient un perméat de microfiltration avec un degré DP de 25 °DP / ml après 1 heure de microfiltration, ce degré reflétant l'activité enzymatique de la solution contenant la β-amylase. La mesure de l'activité enzymatique est déterminée par l'activité diastasique. Cette dernière est exprimée en degré de pouvoir diastasique (°DP), défini comme la quantité d'enzyme contenue dans 0,1 ml d'une solution à 5 % en poids d'un échantillon de préparation d'enzymes suffisante pour réduire 5 ml de liqueur de Fehling, quand ledit échantillon est placé dans 100 ml du substrat pendant 1 h à 20°C.

L'étape de microfiltration est suivie par une étape d'ultrafiltration, réalisée sur le perméat de microfiltration. Elle a pour principal objectif de concentrer ledit perméat et de le débarrasser d'éventuels sels résiduels contaminants, sucres et protéines. Le pilote d'ultrafiltration est équipé de membranes organiques en polysulfone, ayant un seuil de coupure 25 KDa (membranes Alfa Laval). La température de filtration est fixée à 25°C pour limiter au maximum les développements bactériologiques et préserver l'activité enzymatique. La Pression TransMembranaire (PTM) est fixée à 4 bars maximum.

On obtient alors une solution aqueuse de β-amylase qui consiste en le rétentat d'ultrafiltration, ayant une teneur en poids sec de β-amylase égale à 15 % de son poids total.

Différents cocktails, tel qu'indiqué dans les tableaux 1 à 3, ont été testés. Tous les % sont exprimés en % en poids sec de produit, par rapport au poids total de la solution aqueuse. Une fois les préparations réalisées, un dosage enzymatique de chaque échantillon (contenu en pot de 100 ml stériles) est effectué selon la méthode décrite dans la demande de brevet Fr 2 994 440 (mesure de l'activité beta-amylasique). Cette valeur fait objet de référence pour l'ensemble de l'étude. Les différents échantillons sont ensuite placés dans une étuve à température contrôlée : 37°C pendant la période souhaité ; un prélèvement pour mesure de l'activité beta-amylasique résiduelle est alors effectué à différents moments (les jours de prélèvement sont indiqués dans les tableaux 1 à 3). Les résultats figurent dans les tableaux 1 à 3, et sont exprimés comme des % d'activité beta-amylasique résiduelle. On choisit de se placer à 37°C de manière à accélérer les phénomènes qui président à la chute de l'activité enzymatique.

Le tableau 1 bis démontre que le meilleur résultat est obtenu avec le mélange de 40 % de glycérol, 0,2 % de sorbate de potassium et 0,2 % de Na₂CO₃. Il démontre également que, par rapport à d'autres cocktails mettant en oeuvre d'autres ingrédients, c'est bien la solution selon l'invention qui permet de développer le meilleur niveau de stabilité. Il s'agit donc bien d'une sélection non évidente d'ingrédients pour réaliser un cocktail, qui conduit à des résultats surprenants et tout à fait avantageux, en termes de limitation de la perte d'activité enzymatique. Le tableau 1 bis démontre que des cocktails comme décrits dans la revendication 1 de la présente demande permettent de développer des niveaux de stabilité très importants. La plus grande stabilité est par ailleurs obtenue avec le dernier cocktail décrit dans ce tableau, réalisé avec les doses optimales de chaque ingrédient, comme décrit dans la revendication 2 de la présente demande.

Le tableau 2 démontre que le glycérol, utilisé seul, et même à forte dose, ne permet pas d'atteindre le niveau de stabilité satisfaisant. Le tableau 3 démontre que la substitution du glycérol par d'autres sucres ne permet pas elle non plus d'atteindre un niveau de stabilité satisfaisant.

**Tableau 1**

| jours | 50% glycérol + 0,2 % SP | 50% glycérol + 0,2 % SP + 1% Na₂HPO₄ | 40% glycérol + 0,2 % SP + 1% Na₂CO₃ | 40% sorbitol + 0,2 % SP + 1% Na₂HPO₄ | 40% sorbitol + 0,2 % SP + 1 % CaCO₃ | 50% glycérol + 0,2 % SP + 1 % CaCO₃ |
|---|---|---|---|---|---|---|
| 0 | 100 | 100 | 100 | 100 | 100 | 100 |
| 20 | 98 | 98 | | | | |
| 30 | | | 72 | | | |
| 34 | 89 | 89 | | 77 | 85 | 92 |
| 60 | | | 60 | | | |
| 72 | 48 | 70 | 75 | 66 | 69 | 70 |
| 90 | | 45 | 50 | 44 | 45 | 47 |

**Tableau 1 bis**

| jours | 60% glycérol + 0,2 % SP + 0,4% Na₂CO₃ | 40% glycérol + 1 % SP+ 0,4% Na₂CO₃ | 40% glycérol + 0,2 % SP + 0,4% Na₂CO₃ | 40% glycérol + 0,4 % SP + 0,2% Na₂CO₃ | 40% glycérol + 0,2 % SP + 0,2% Na₂CO₃ |
|---|---|---|---|---|---|
| 0 | 100 | 100 | 100 | 100 | 100 |
| 72 | 74 | 73 | 76 | 76 | 80 |
| 90 | 49 | 48 | 54 | 54 | 60 |

**Tableau 2***

| | 0% glycérol | 30% glycérol | 40% glycérol | 50% glycérol |
|---|---|---|---|---|
| 0 | 100 | 100 | 100 | 100 |
| 30 | 0 | 53 | 65 | 69 |
| 60 | 0 | 27 | 44 | 56 |
| 90 | 0 | 7 | 16 | 28 |

**Tableau 3**

| jours | 50% glucose | 10% glycerol + 30% glucose | 20% glycerol + 20% glucose | 40% glucose + 0,5% Na₂HPO₄ | 40% glucose + 3% NaCl | 40% maltose | 40% mélange (45% glucose, 10% fructose, 45% maltose) |
|---|---|---|---|---|---|---|---|
| 0 | 100 | 100 | 100 | 100 | 100 | 100 | 100 |
| 30 | 66 | 53 | 61 | 82 | 42 | 46 | 39 |
| 60 | 43 | 35 | 37 | 43 | 15 | 30 | 20 |
| 90 | 28 | 22 | 22 | 22 | 7 | | |

| | | | | | | | |
|---|---|---|---|---|---|---|---|
| SP : sorbate de potassium * on note par ailleurs la formation d'un dépôt insoluble important dans le cas du carbonate de calcium | | | | | | | |

### Fabrication de sirops de maltose

On réalise ensuite 2 essais, portant sur la fabrication de sirops de maltose à partir de deux solutions aqueuses de beta-amylase stabilisées par un cocktail selon l'invention ou par un cocktail hors invention, ces 2 solutions ayant été maintenues 90 jours à 25 °C avant d'être utilisées.

Un lait d'amidon, à une matière sèche de 31 %, est liquéfié de manière classique à l'aide de 0,2 % d'une alpha-amylase (TERMAMYL120L commercialisée par la société NOVOZYME) à un pH de 5,7 à 6,5 jusqu'à un DE légèrement environ égal à 6.

On chauffe ensuite le milieu réactionnel pendant quelques secondes à 140 °C de manière à inhiber l'alpha-amylase, puis on ajuste le pH entre 5 et 5,5 et la température à 55 °C.

La saccharification est conduite à une matière sèche de 35 %, ou légèrement inférieure, en présence de pullulanase (PULLUZYME 750L commercialisée par la société ABM) et d'alpha-amylase maltogénique (MALTOGENASE 4000L commercialisée par la société NOVOZYME) et d'une solution aqueuse de beta-amylase, à des doses égales à 0,1 % sur matière sèche.

La solution aqueuse de beta-amylase consiste en le rétentat d'ultrafiltration, ayant une teneur en poids sec de alpha-amylase égale à 15 % de son poids total, comme décrit dans l'exemple précédent.

Dans un premier test hors invention, cette solution a été stabilisée avec le cocktail selon la deuxième colonne du tableau 1 (50% glycérol + 0,2 % SP + 1% Na₂HPO₄). La solution est restée à une température de 25°C pendant 90 jours avant d'être utilisée comme indiquée ci-dessus.

Dans un second test selon l'invention, cette solution a été stabilisée avec le cocktail selon la dernière colonne du tableau 1 bis (40% glycérol + 0,2 % SP + 0,2% Na₂CO₃). La solution est restée à une température de 25°C pendant 90 jours avant d'être utilisée comme indiquée ci-dessus.

Pour ces 2 tests, la saccharification, qui dure environ 72 heures, donne un hydrolysat montrant la composition suivante :
Hors invention :
   DP1 : 2 %, DP2 : 77,9 % DP3 : 5,6 %
Selon l'invention :
   DP1 : 5 %, DP2 : 88 % DP3 < 1,5 %

### Fabrication de sirops de maltose

On réalise ensuite 4 essais, portant sur la fabrication de sirops de maltose à partir de 4 solutions aqueuses de beta-amylase stabilisées. 3 essais selon l'invention et 1 essai de référence sont réalisés en utilisant des solutions stabilisées ayant été stockées 90 jours à 25 °C avant d'être utilisées.

Un lait d'amidon, à une matière sèche de 31 %, est liquéfié de manière classique à l'aide de 0,2 % d'une alpha-amylase (TERMAMYL120L commercialisée par la société NOVOZYMES) à un pH de 5,7 à 6,5 jusqu'à un DE environ égal à 6.

On chauffe ensuite le milieu réactionnel pendant quelques secondes à 140 °C de manière à inhiber l'alpha-amylase, puis on ajuste le pH entre 5 et 5,5 et la température à 55 °C.

La saccharification est conduite à une matière sèche de 35 %, ou légèrement inférieure, en présence de pullulanase (PULLUZYME 750L commercialisée par la société ABM) et d'alpha-amylase maltogénique (MALTOGENASE 4000L commercialisée par la société NOVOZYMES) et d'une solution aqueuse de beta-amylase, à des doses égales à 0,1 % sur matière sèche.

La solution aqueuse de beta-amylase consiste en le rétentat d'ultrafiltration, ayant une teneur en poids sec de beta-amylase égale à 15 % de son poids total, comme décrit dans l'exemple précédent.

Dans un premier test hors invention (CP), cette solution a été stabilisée avec le cocktail selon la deuxième colonne du tableau 1 (50% glycérol + 0,2 % SP + 1% Na₂HPO₄). La solution est restée à une température de 25°C pendant 90 jours avant d'être utilisée comme indiquée ci-dessus.

Dans un deuxième test selon l'invention (EX1), cette solution a été stabilisée avec le cocktail selon la dernière colonne du tableau 1 bis (40% glycérol + 0,2 % SP + 0,2% Na₂CO₃). La solution est restée à une température de 25°C pendant 90 jours avant d'être utilisée comme indiquée ci-dessus.
Dans un troisième test selon l'invention (EX2), cette solution a été stabilisée avec le cocktail selon la quatrième colonne du tableau 1 bis (40% glycérol + 0,4 % SP + 0,2% Na₂CO₃). La solution est restée à une température de 25°C pendant 90 jours avant d'être utilisée comme indiquée ci-dessus.

Dans un quatrième test selon l'invention (EX3), cette solution a été stabilisée avec le cocktail selon la troisième colonne du tableau 1 (40% glycérol + 0,2 % SP + 1% Na₂CO₃). La solution est restée à une température de 25°C pendant 90 jours avant d'être utilisée comme indiquée ci-dessus.

Pour ces tests, la saccharification, qui dure environ 72 heures, donne un sirop de maltose montrant pour chacun des exemples les compositions suivantes :
Sirop de maltose CP :
   glucose : 2 %, maltose : 77,9 % maltotriose : 5,6 %
Sirop de maltose EX1 :
   glucose : 5 %, maltose : 88 % maltotriose : < 1,5 %
Sirop de maltose EX2 :
   glucose : 3,2 % - maltose : 82,1 % - maltotriose: 3,7 %
Sirop de maltose EX3 :
   glucose : 2,8 % - maltose : 81 % - maltotriose : 4,6 %

## Revendications

1. Procédé de stabilisation d'une solution aqueuse de β-amylase obtenue à partir d'une fraction soluble de plante amidonnière, comprenant au moins une étape d'introduction dans ladite solution aqueuse de β-amylase de :
a) 0,05 à 0,5 % de sorbate de potassium ;
b) 30 à 50 % de glycérol ;
c) 0,05 à 0,5 % carbonate de sodium
ces % étant exprimés en % en poids sec de chaque constituant par rapport au poids total de ladite solution aqueuse.

2. Procédé selon la revendication précédente, **caractérisé en ce qu'**on introduit dans ladite solution aqueuse de β-amylase :
a) de 0,1 à 0,3 %, et préférentiellement environ 0,2 % de sorbate de potassium ;
b) de 35 à 45 %, et préférentiellement environ 40 % de glycérol ;
c) de 0,1 à 0,3 %, et préférentiellement environ 0,2 % de carbonate de sodium ;
ces % étant exprimés en % en poids sec de chaque constituant par rapport au poids total de ladite solution aqueuse.

3. Procédé selon une des revendications précédentes, **caractérisé en ce que** la solution aqueuse présente une teneur en poids sec de β-amylase comprise entre 5 et 20 %, préférentiellement entre 10 et 20 %, très préférentiellement égale à environ 15 % de son poids total.

4. Procédé selon une des revendications précédentes, **caractérisé en ce que** le sorbate de potassium, le glycérol et le carbonate de sodium sont sous forme de solutions aqueuses.

5. Procédé selon la revendication précédente, dans lequel la solution aqueuse de β-amylase obtenue par les étapes consistant à :
- fournir une fraction soluble de plantes amidonnières ;
- réaliser sur ladite fraction soluble une étape de microfiltration afin d'obtenir un perméat de microfiltration ;
réaliser sur le perméat de microfiltration une étape d'ultrafiltration afin d'obtenir un rétentat d'ultrafiltration.

6. Utilisation, en vue de maintenir l'activité enzymatique de β-amylase dans une solution aqueuse, de :
a) 0,05 à 0,5 % de sorbate de potassium ;
b) 30 à 50 % de glycérol ; et
c) 0,05 à 0,5 % de carbonate de sodium,
ces % étant exprimés en % en poids sec de chaque constituant par rapport au poids total de ladite solution aqueuse.

7. Solution aqueuse de β-amylase, contenant :
a) de 0,05 à 0,5 % de sorbate de potassium ;
b) de 30 à 50 % de glycérol ; et
c) de 0,05 à 0,5 % de carbonate de sodium,
ces % étant exprimés en % en poids sec de chaque constituant par rapport au poids total de ladite solution aqueuse.

8. Solution aqueuse selon la revendication précédente, contenant :
a) de 0,1 à 0,3 %, et préférentiellement environ 0,2 % de sorbate de potassium ;
b) de 35 à 45 %, et préférentiellement environ 40 % de glycérol ;
c) 0,1 à 0,3 %, et préférentiellement environ 0,2 % de carbonate de sodium ;
ces % étant exprimés en % en poids sec de chaque constituant par rapport au poids total de ladite solution aqueuse.

9. Solution aqueuse selon une des revendications 7 ou 8, présentant une teneur en poids sec de β-amylase comprise entre 5 et 20 %, préférentiellement entre 10 % et 20 %, très préférentiellement environ 15 % de son poids total.

10. Utilisation de la solution aqueuse selon une des revendications 7 à 9, comme additif alimentaire, comme agent digestif, pour la production d'édulcorants, en pharmacie pour la production de vaccins, et enfin pour la production de maltose et de sirops enrichis en maltose.

## Patentansprüche

1. Verfahren zum Stabilisieren einer wässrigen β-Amylase-Lösung, erhalten aus einer löslichen Stärkepflanzen-Fraktion, umfassend mindestens einen Schritt des Einbringens in die wässrige β-Amylase-Lösung von:
a) 0,05 bis 0,5 % Kaliumsorbat,
b) 30 bis 50 % Glycerin,
c) 0,05 bis 0,5 % Natriumcarbonat,
wobei diese % ausgedrückt sind als % Trockengewicht jedes Bestandteils in Bezug auf das Gesamtgewicht der wässrigen Lösung.

2. Verfahren nach dem vorhergehenden Anspruch, **dadurch gekennzeichnet, dass** man in die wässrige β-Amylase-Lösung einbringt:
a) 0,1 bis 0,3 %, und bevorzugt etwa 0,2 % Kaliumsorbat,
b) 35 bis 45 %, und bevorzugt etwa 40 % Glycerin,
c) 0,1 bis 0,3 %, und bevorzugt etwa 0,2 % Natriumcarbonat,
wobei diese % ausgedrückt sind als % Trockengewicht jedes Bestandteils in Bezug auf das Gesamtgewicht der wässrigen Lösung.

3. Verfahren nach einem der vorhergehenden Ansprüche, **dadurch gekennzeichnet, dass** die wässrige Lösung einen Trockengewicht-Gehalt von β-Amylase zwischen 5 und 20 % aufweist, vorzugsweise zwischen 10 und 20 %, sehr bevorzugt gleich ungefähr 15 % ihres Gesamtgewichts.

4. Verfahren nach einem der vorhergehenden Ansprüche, **dadurch gekennzeichnet, dass** das Kaliumsorbat, das Glycerin und das Natriumcarbonat in Form von wässrigen Lösungen vorliegen.

5. Verfahren nach dem vorhergehenden Anspruch, wobei die wässrige β-Amylase-Lösung erhalten wird durch die Schritte:
- Bereitstellen einer löslichen Fraktion von Stärkepflanzen,
- Durchführen eines Mikrofiltrationsschritts an der löslichen Fraktion, um ein Permeat der Mikrofiltration zu erhalten, Durchführen eines Ultrafiltrationsschritts an dem Permeat der Mikrofiltration, um ein Retentat der Ultrafiltration zu erhalten.

6. Verwendung, um die enzymatische Aktivität von β-Amylase in einer wässrigen Lösung aufrechtzuerhalten, von:
a) 0,05 bis 0,5 % Kaliumsorbat
b) 30 bis 50 % Glycerin und
c) 0,05 bis 0,5 % Natriumcarbonat,
wobei diese % ausgedrückt sind als % Trockengewicht jedes Bestandteils in Bezug auf das Gesamtgewicht der wässrigen Lösung.

7. Wässrige β-Amylase-Lösung, enthaltend:
a) 0,05 bis 0,5 % Kaliumsorbat,
b) 30 bis 50 % Glycerin und
c) 0,05 bis 0,5 % Natriumcarbonat,
wobei diese % ausgedrückt sind als % Trockengewicht jedes Bestandteils in Bezug auf das Gesamtgewicht der wässrigen Lösung.

8. Wässrige Lösung nach dem vorhergehenden Anspruch, enthaltend:
a) 0,1 bis 0,3 %, und bevorzugt etwa 0,2 % Kaliumsorbat,
b) 35 bis 45 %, und bevorzugt etwa 40 % Glycerin,
c) 0,1 bis 0,3 %, und bevorzugt etwa 0,2 % Natriumcarbonat,
wobei diese % ausgedrückt sind als % Trockengewicht jedes Bestandteils in Bezug auf das Gesamtgewicht der wässrigen Lösung.

9. Wässrige Lösung nach einem der Ansprüche 7 oder 8, welche einen Trockengewicht-Gehalt von β-Amylase zwischen 5 und 20 % aufweist, vorzugsweise zwischen 10 % und 20 %, sehr bevorzugt etwa 15 % ihres Gesamtgewichts.

10. Verwendung der wässrigen Lösung nach einem der Ansprüche 7 bis 9, als Lebensmittelzusatzstoff, als Verdauungsmittel, zur Herstellung von Süßstoffen, in der Pharmazie zur Herstellung von Impfstoffen, und schließlich zur Herstellung von Maltose und von mit Maltose angereicherten Sirupen.

## Claims

1. A method for stabilizing an aqueous solution of β-amylase obtained from a soluble fraction of starch plants, comprising at least one step of introducing, into said aqueous solution of β-amylase:
a) 0.05 to 0.5% of potassium sorbate;
b) 30 to 50% of glycerol;
c) 0.05 to 0.5% of sodium carbonate;
these % being expressed as % by dry weight of each constituent relative to the total weight of said aqueous solution.

2. The method as claimed in the preceding claim, **characterized in that** the following are introduced into said aqueous solution of β-amylase:
a) from 0.1 to 0.3%, and preferentially approximately 0.2% potassium sorbate;
b) from 35 to 45%, and preferentially approximately 40% glycerol;
c) 0.1 to 0.3%, and preferentially approximately 0.2% sodium carbonate;
these % being expressed as % by dry weight of each constituent relative to the total weight of said aqueous solution.

3. The method as claimed in one of the preceding claims, **characterized in that** the aqueous solution has a content by dry weight of β-amylase of between 5 and 20%, preferentially between 10 and 20%, very preferentially equal to approximately 15% of the total weight thereof.

4. The method as claimed in one of the preceding claims, **characterized in that** the potassium sorbate, the glycerol and the sodium carbonate are in the form of aqueous solutions.

5. The method as claimed in the preceding claim, wherein the aqueous solution of β-amylase is obtained by the steps consisting in:
- providing a soluble fraction of starch plants;
- carrying out a microfiltration step on said soluble fraction, in order to obtain a microfiltration permeate;
- carrying out an ultrafiltration step on the microfiltration permeate, in order to obtain an ultrafiltration retentate.

6. The use, with a view to maintaining the enzymatic activity of β-amylase in an aqueous solution, of:
a) 0.05 to 0.5% of potassium sorbate;
b) 30 to 50% of glycerol;
c) 0.05 to 0.5% of sodium carbonate;

7. An aqueous solution of β-amylase, containing:
a) 0.05 to 0.5% of potassium sorbate;
b) 30 to 50% of glycerol;
c) 0.05 to 0.5% of sodium carbonate;

8. The aqueous solution as claimed in the preceding claim, containing:
a 0.1 to 0.3%, and preferentially approximately 0.2% potassium sorbate;
b) from 35 to 45%, and preferentially approximately 40% glycerol;
c) from 0.1 to 0.3%, and preferentially approximately 0.2% sodium carbonate;
these % being expressed as % by dry weight of each constituent relative to the total weight of said aqueous solution.

9. The aqueous solution as claimed in either of claims 7 and 8, having a content by dry weight of β-amylase of between 5 and 20%, preferentially between 10 and 20%, very preferentially approximately 15% of the total weight thereof.

10. The use of the aqueous solution as claimed in one of claims 7 to 9, as food additive, as digestive agent, for the production of sweeteners, in pharmacy for the production of vaccines, and finally for the production of maltose and of maltose-enriched syrups.
